# EUROPEAN PATENT APPLICATION

(11) **EP 3 760 251 A1**
(43) Date of publication of application: **06.01.2021**
(21) Application number: 19777665.1
(22) Date of filing: 27.03.2019
(51) Int. Cl.: A61M 5/158, A61M 5/142, A61M 5/145

(54) **MEDICATING APPLIANCE AND LIQUID MEDICINE ADMINISTERING SYSTEM**

(30) Priority: 30.03.2018 JP 2018067426
(71) Applicant: TERUMO Kabushiki Kaisha, Tokyo 151-0072 (JP)
(72) Inventor: ARINOBE, Manabu, Ashigarakami-gun, Kanagawa 259-0151 (JP); HYAKKAN, Yusuke, Ashigarakami-gun, Kanagawa 259-0151 (JP); OKIHARA, Hitoshi, jinomiya-shi, Shizuoka 418-0004 (JP)
(74) Representative: TBK
(86) International application number: PCT/JP2019/013309
(87) International publication number: WO 2019/189434

(57) **Abstract**

To provide an administering instrument capable of preventing a needle tube from coming out of a living body during administration of a medicinal solution and a medicinal solution administering system including the administering instrument and a medicinal solution administering device.

An administering instrument 200 includes a first end part 241 connected to a puncture part 230 holding the needle tube, a second end part 242 connected to a connector 210 connectable to the medicinal solution administering device, and a tube body 243 communicating from the first end part to the second end part, a tube configured to supply the medicinal solution from a lumen of a medicinal solution container to the needle tube via a communication part of the connector and a communication passage of the puncture part, and the tube body has a total length longer than a straight-line distance d1 between the first end part and the second end part.

## Description

### Technical Field

The present invention relates to an administering instrument and a medicinal solution administering system including the administering instrument and a medicinal solution administering device.

### Background Art

A syringe pump-type medicinal solution administering device is conventionally known which administers a medicinal solution filled in a medicinal solution container to a living body by a pressing action of a plunger. Further, in administering a medicinal solution with a medicinal solution administering device of this type, an administering instrument is sometimes used which includes a puncture part (cannula housing) connected to the medicinal solution administering device via a predetermined connector and tube.

The puncture part provided in the administering instrument has a contact surface (bottom surface) that comes into contact with a body surface of a user. Further, the puncture part holds a needle tube arranged so as to project from the contact surface. The needle tube held by the puncture part supplies a medicinal solution fed from the medicinal solution container via a tube or the like to the living body with the needle tube puncturing the living body.

### Citation List

### Patent Literature

Patent Literature 1: JP 2009-233388 A

### Summary of Invention

### Technical Problem

In the medicinal solution administering system, the administering instrument is attached to the body surface of the user via the contact surface of the puncture part while the medicinal solution is administered to the living body. Similarly, the medicinal solution administering device is attached to the body surface of the user through the contact surface (bottom surface) of the housing in which each constituent member of the medicinal solution administering device is assembled. Therefore, while the medicinal solution is administered to the user, the puncture part of the administering instrument and the medicinal solution administering device are attached to the body surface of the user with the puncture part and the medicinal solution administering device connected to each other via the tube. For example, when the user moves such as twisting his/her body, the puncture part is pulled toward the medicinal solution administering device via the tube. Thereby, a force that lifts the puncture part from the body surface acts on the puncture part, which sometimes causes the puncture part to come off the body surface. Further, this possibly causes the needle tube held by the puncture part to come out of the living body.

Particularly, in a case where the medicinal solution administering device and the puncture part are attached to a curved body surface (for example, abdomen, thigh, and so on) or where a thin puncture part is attached to the body surface and then a deviation (difference) in the height direction occurs between a position of the contact surface of the puncture part and a position of the contact surface of the medicinal solution administering device, a force that lifts the puncture part from the body surface via the tube is likely to act on the puncture part, which increases the possibility that the needle tube is removed.

The present invention has been made in view of the above issues, and an object of the present invention is to provide an administering instrument that is capable of preventing a needle tube from coming out of a living body during administration of a medicinal solution, and to provide a medicinal solution administering system including the administering instrument and a medicinal solution administering device.

### Solution to Problem

An administering instrument according to the present invention is an administering instrument connectable to a medicinal solution administering device including a medicinal solution container in which a medicinal solution is filled. The administering instrument includes a connector that includes a communication part capable of communicating with a lumen of the medicinal solution container and is connectable to the medicinal solution administering device; a needle tube that punctures a living body; a puncture part including a contact surface that is provided in a lower end to contact a living body surface of the living body, a needle holding part that is provided above the contact surface to hold the needle tube so that a tip of the needle tube projects from the contact surface, and a communication passage communicating with a lumen of the needle tube; and a tube that includes a first end part connected to the puncture part, a second end part connected to the connector, and a tube body communicating from the first end part to the second end part and is configured to supply the medicinal solution from the lumen of the medicinal solution container to the needle tube via the communication part of the connector and the communication passage of the puncture part. The tube body has a total length longer than a straight-line distance between the first end part and the second end part.

Further, a medicinal solution administering system according to the present invention includes a medicinal solution administering device having a medicinal solution container, a housing that holds the medicinal solution container, and a plunger that pushes out the medicinal solution in the medicinal solution container to the tube.

### Advantageous Effects of Invention

According to the administering instrument and the medicinal solution administering system of the present invention, it is possible to prevent the needle tube from coming out of a living body during administration of a medicinal solution.

### Brief Description of Drawings

Fig. 1 is a side view of a medicinal solution administering system according to a first embodiment of the present invention.
Fig. 2 is a diagram schematically illustrating an example of the use of a medicinal solution administering system according to the first embodiment.
Fig. 3 is a sectional view of a medicinal solution administering device and an administering instrument according to the first embodiment.
Fig. 4 is an enlarged side view of a puncture part of an administering instrument according to the first embodiment.
Fig. 5 is an enlarged plan view of a puncture part of an administering instrument according to the first embodiment.
Fig. 6 is an enlarged side view of a puncture part of an administering instrument according to a second embodiment of the present invention.
Fig. 7 is an enlarged plan view of a puncture part of an administering instrument according to the second embodiment.

### Description of Embodiments

Hereinafter, embodiments of the present invention are described with reference to the accompanying drawings. The following description does not limit the technical scope described in the claims and the meaning of terms used in the claims. Further, the dimensional ratios in the drawings are exaggerated for convenience of description, and may differ from the actual ratios.

### First Embodiment

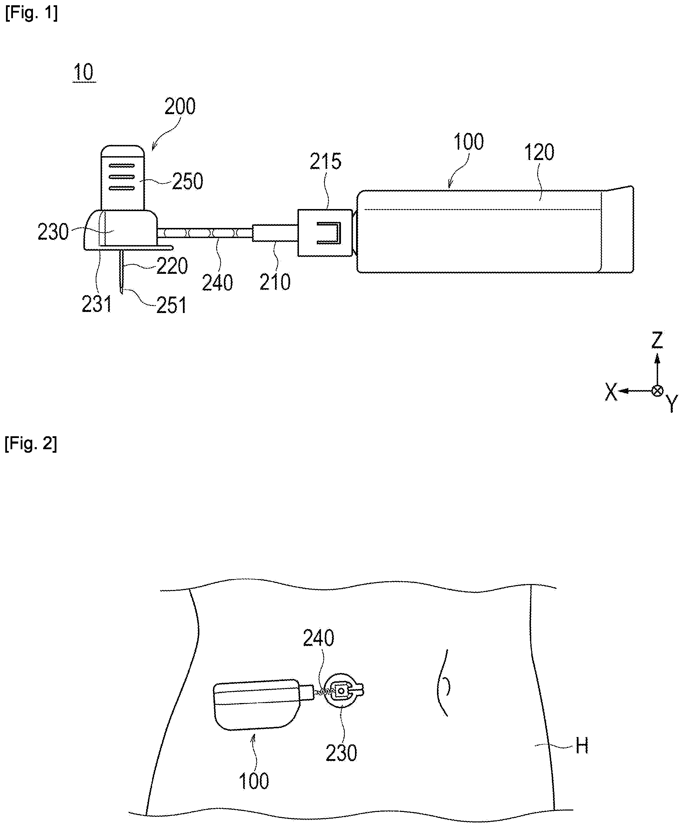

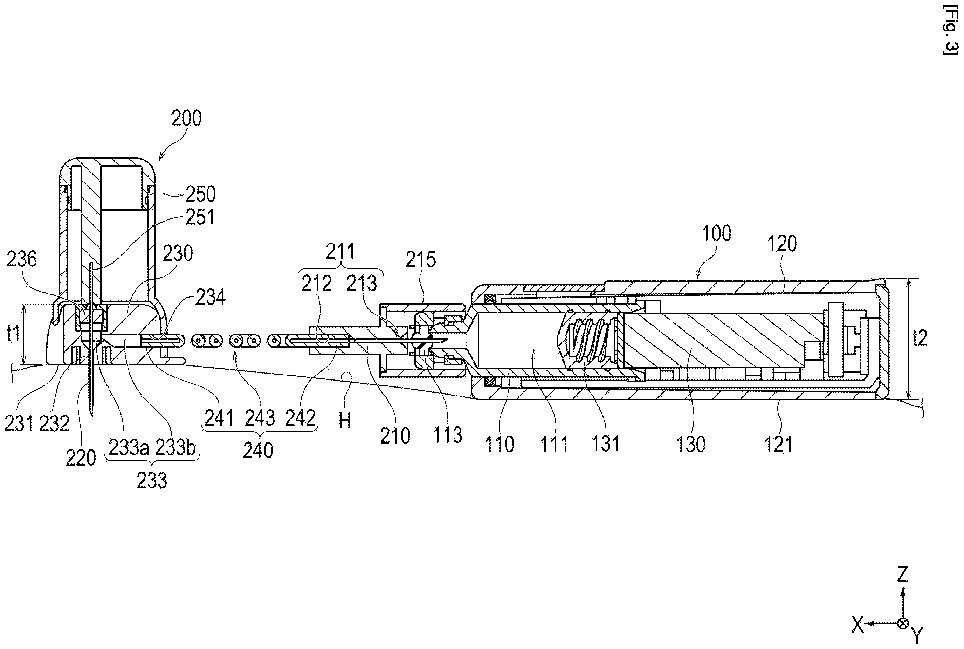

Figs. 1 to 5 are diagrams for explaining a medicinal solution administering system 10, a medicinal solution administering device 100, and an administering instrument 200 according to the first embodiment. In the diagrams, an arrow X indicates a "longitudinal direction (extending direction of a tube 240)" of the medicinal solution administering device 100 and the administering instrument 200, an arrow Y indicates a "width direction (depth direction)" of the medicinal solution administering device 100 and the administering instrument 200, and an arrow Z indicates a "height direction (thickness direction of a puncture part 230)" of the medicinal solution administering device 100 and the administering instrument 200. Further, a sectional view illustrated in Fig. 3 is a longitudinal sectional view (sectional view taken along the section X-Z) of the medicinal solution administering device 100 and the administering instrument 200.

### Medicinal Solution Administering System

The medicinal solution administering system 10 is used to administer a medicinal solution to a living body. As illustrated in Fig. 1, the medicinal solution administering system 10 includes the medicinal solution administering device 100 and the administering instrument 200.

As illustrated in Figs. 2 and 3, when the medicinal solution administering system 10 is used to administer a medicinal solution, the medicinal solution administering device 100 and the puncture part 230 of the administering instrument 200 are attached to a body surface H of a user. The location of the body of the user to which the medicinal solution administering device 100 and the puncture part 230 are attached is not particularly limited, but is the abdomen or thigh for example.

The medicinal solution administering system 10 can continuously administer, for example, a medicinal solution (not shown) filled in a medicinal solution container 110 of the medicinal solution administering device 100 to a living body over a relatively long time (for example, a few minutes to a few hours) by a pressing action of a plunger 130. The medicinal solution administering system 10 may intermittently administer the medicinal solution to the living body.

### Medicinal Solution Administering Device

As illustrated in Fig. 3, the medicinal solution administering device 100 includes the medicinal solution container 110 in which a medicinal solution is filled, a housing 120 for holding the medicinal solution container therein, and the plunger 130 for pushing out the medicinal solution held in the medicinal solution container 110 to the tube 240 described later.

The medicinal solution container 110 has a cylindrical body including a lumen 111 in which the medicinal solution is filled. A tip of the plunger 130 is inserted into the medicinal solution container 110. A gasket 131 is disposed at the tip of the plunger 130. The gasket 131 can be made of, for example, a rubber material or a resin material such as an elastomer. The outer peripheral portion of the gasket 131 liquid-tightly contacts the inner peripheral surface of the medicinal solution container 110, which liquid-tightly seals a proximal end side of the gasket 131.

The medicinal solution container 110 is constituted by a so-called pre-filled type medicinal solution container. The medicinal solution is therefore filled, in advance, in the lumen 111 of the medicinal solution container 110. Examples of the medicinal solution include a protein preparation, a narcotic analgesic, and a diuretic.

A sealing member 113 for preventing leakage of the medicinal solution is provided in a tip opening (discharge port) of the medicinal solution container 110. The tip opening of the medicinal solution container 110 is disposed so as to project to the outside of the housing 120.

The plunger 130 is configured to be movable forward with respect to the medicinal solution container 110 toward the tip side (left side in Fig. 3) of the medicinal solution container 110 by an advancing mechanism (not shown). The plunger 130 moves forward with respect to the medicinal solution container 110 to push out the medicinal solution to the tube 240 from the lumen 111 of the medicinal solution container 110.

The housing 120 accommodates therein a battery for supplying power necessary for each operation of the medicinal solution administering device 100, a substrate to which a controller unit for controlling a motor is attached, a drive mechanism for driving the advancing mechanism, and so on (all are not shown).

The medicinal solution administering device 100 is configured as a patch type device attached to the body surface (skin) H of the user and used. The housing 120 of the medicinal solution administering device 100 has, on a contact surface (bottom surface) 121, a sheet-like adhesive part (not shown) that can be adhered to the body surface. In the initial state before the medicinal solution administering device 100 is attached to the user, a peelable protective sheet is attached to an adhesive surface of the adhesive part.

### Administering Instrument

As illustrated in Figs. 1 and 2, the administering instrument 200 is configured to be connectable to the medicinal solution administering device 100.

As outlined with reference to Figs. 3 to 5, the administering instrument 200 includes a connector 210, a needle tube 220 to puncture a living body, the puncture part (cannula housing) 230, the tube 240, and a puncture auxiliary tool 250 for assisting the needle tube 220 in puncturing the living body.

As illustrated in Fig. 3, the connector 210 has a communication part 211 that can communicate with the lumen 111 of the medicinal solution container 110. The connector 210 is configured to be connectable to the medicinal solution administering device 100 via a mounting part 215 fixed to the connector 210. As illustrated in Fig. 3, the mounting part 215 is externally fitted around the tip of the medicinal solution container 110 projecting to the outside of the housing 120, so that the mounting part 215 can be connected to the medicinal solution administering device 100.

As illustrated in Fig. 3, the communication part 211 of the connector 210 includes a first connection part 213 connected to a second end part 242 of the tube 240 and a second connection part 214 communicating with the lumen 111 of the medicinal solution container 110.

The first connection part 213 of the communication part 211 is constituted by an insertion hole into which the second end part 242 of the tube 240 is inserted and fixed. The second connection part 214 of the communication part 211 is constituted by a connection needle tube 212 having a needle tip capable of puncturing the sealing member 113. An end on the connector 210 side of the connection needle tube 212 is fixed to the connector 210.

The tube 240 and the lumen 111 of the medicinal solution container 110 communicate with each other in a state (state illustrated in Fig. 3) where the connector 210 is connected to the medicinal solution administering device 100 through the mounting part 215 and where the needle tip of the connection needle tube 212 penetrates the sealing member 113 of the medicinal solution administering device 100.

As illustrated in Fig. 3, the puncture part 230 includes a contact surface 231 that is a lower end of the puncture part 230 and comes into contact with the body surface H of the user, a needle holding part 232 that is disposed above the contact surface 231 and holds the needle tube 220 so that a tip (lower end) 221 of the needle tube 220 projects from the contact surface 231, a communication passage 233 that communicates with a lumen of the needle tube 220, and a fixing part 234 that is disposed above the contact surface 231 and fixes a first end part 241 of the tube 240 to the puncture part 230.

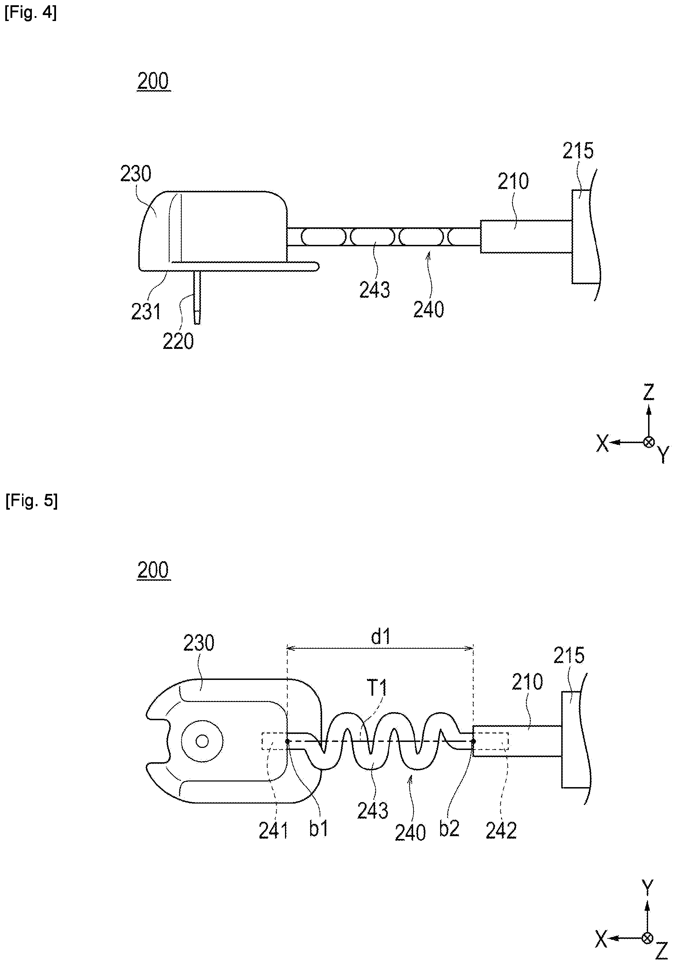

The tube 240 is configured so that a medicinal solution can be supplied from the lumen 111 of the medicinal solution container 110 to the needle tube 220 via the communication part 211 of the connector 210 and the communication passage 233 of the puncture part 230. As illustrated in Figs. 3 and 5, the tube 240 includes the first end part 241 connected to the puncture part 230, the second end part 242 connected to the connector 210, and a tube body 243 extending from the first end part 241 to the second end part 242.

The "first end part 241" and the "second end part 242" in the present embodiment do not specify the exact positions of the tube 240, but mean a certain range of a part of the tube 240 fixed to (inserted into) the puncture part 230 and a certain range of a part of the tube 240 fixed to (inserted into) the connector 210. Further, the "tube body 243" in the present embodiment means a part of the tube 240 exposed from the puncture part 230 and the connector 210 in the plan view illustrated in Fig. 5.

As illustrated in Fig. 5, the tube body 243 has a total length longer than a straight-line distance d1 between the first end part 241 and the second end part 242 (length of the tube body 243 straightened out). Further, at least a part of the tube body 243 extends in a bellows shape.

The "bellows shape" means, in the plan view illustrated in Fig. 5, a two-dimensionally curved or bent shape in the Y-direction of Fig. 5 with respect to a straight line T1 connecting a boundary b1 between the first end part 241 and the tube body 243 (an end on the first end part 241 side of the tube body 243) and a boundary b2 between the second end part 242 and the tube body 243 (an end on the second end part 242 side of the tube body 243). In other words, the tube 240 has a shape in which at least a part of the tube body 243 is bent in the Y-direction.

The length, in the Y-direction, of the curved or bent part (bellows part) of the tube body 243, the number of repetitions of curvature or bending in the direction in which the tube body 243 extends, the position at which the bellows part of the tube body 243 is formed, and so on are not particularly limited, and can be modified suitably.

The length of the tube body 243 means a length of the entirety of the tube 240 stretched to a nearly straight shape (the maximum length of the tube body 243). Further, the straight-line distance d1 between the first end part 241 and the second end part 242 corresponds to a length of the straight line connecting the boundary b1 and the boundary b2.

The tube 240 can be formed to have a total length (length in a state where the first end part 241, the second end part 242, and the tube body 243 are straightened out) of, for example, 20 mm to 200 mm, the first end part 241 of the tube 240 (length of a part inserted into the puncture part 230) can be formed to be, for example, 2 mm to 20 mm, the second end part 242 of the tube 240 (length of a part inserted into the connector 210) can be formed to be, for example, 2 mm to 20 mm, and the tube body 243 of the tube 240 can be formed to have a total length (length in a state where the tube body 243 is straightened out) of, for example, 15 mm to 196 mm. Further, a part with a bellows shape of the tube body 243 can be formed to have a length of, for example, 15 mm to 196 mm in a state where the part is straightened out.

The straight-line distance d1 between the first end part 241 and the second end part 242 can be 10 mm to 150 mm, for example. The total length of the tube body 243 is preferably 1.3 to 10 times longer than the straight-line distance d1.

The tube 240 can be made of, for example, a hollow member made of resin having flexibility. Specific examples of the constituent material of the tube 240 include polyethylene, polypropylene, polybutadiene, polyolefin such as ethylene-propylene copolymer and ethylene-vinyl acetate copolymer, thermoplastic resin such as flexible polyvinyl chloride, various rubbers such as silicone rubber and latex rubber, and various elastomers such as polyurethane elastomer, polyamide elastomer, and polyester elastomer.

As illustrated in Fig. 3, in order to supply the medicinal solution to the user, the puncture auxiliary tool 250 is attached to the puncture part 230. The puncture auxiliary tool 250 holds an introducer needle (inner needle) 251. The introducer needle 251 is inserted into the communication passage 233 of the puncture part 230 and the lumen of the needle tube 220 in a state where the puncture auxiliary tool 250 is attached to the puncture part 230. In this state, a tip of the introducer needle 251 projects from the tip of the needle tube 220. The user can insert the needle tube 220 into the living body by puncturing the living body with the needle tube 220 with the introducer needle 251 inserted into the needle tube 220 while preventing the needle tube 220 from being broken.

The puncture auxiliary tool 250 is detached from the puncture part 230 after the needle tube 220 punctures the living body. The introducer needle 251 is removed from the lumen of the needle tube 220 when the puncture auxiliary tool 250 is detached from the puncture part 230. As illustrated in Fig. 3, the tip of the needle tube 220 is formed to be a pointed end so that the tip can be inserted into the living body, and the needle tube 220 is formed to have a taper shape toward a proximal end in the vicinity thereof so that the medicinal solution flows efficiently from the communication passage 233 to the lumen of the needle tube 220.

When the introducer needle 251 is removed, in the needle tube 220, the lumen of the needle tube 220 communicates with the communication passage 233 of the puncture part 230. Further, the lumen of the needle tube 220 communicates with the lumen of the tube 240 via the communication passage 233. The administering instrument 200 administers, to the living body, the medicinal solution fed from the medicinal solution container 110 of the medicinal solution administering device 100 via the tube 240 with the needle tube 220 puncturing the living body. In the vicinity of the proximal end (near the upper end) of the communication passage 233, a sealing member 236 is disposed to prevent the leakage of the medicinal solution from the proximal end of the communication passage 233.

The introducer needle 251 can be constituted by a metal needle, for example. Further, the needle tube 220 can be constituted by, for example, a tubular member (cannula) made of resin.

The puncture auxiliary tool 250 can be prepared in a state of being attached in advance to the puncture part 230, for example, before the use of the medicinal solution administering system 10. In the present embodiment, the needle tube 220 is constituted as a double needle that punctures the living body with the introducer needle 251 inserted. For example, instead of such a double needle structure, the needle tube 220 may have a structure that enables the living body to be punctured only with the needle tube 220. In the case of such a structure, the needle tube 220 is preferably configured by a metal needle.

As illustrated in Fig. 3, the communication passage 233 of the puncture part 230 includes a first channel 233a extending from the proximal end of the needle tube 220 along an axis of the needle tube 220 and a second channel 233b communicating the first channel 233a and the first end part 241 of the tube 240 with each other.

As illustrated in Fig. 3, the fixing part 234 of the puncture part 230 is constituted by an insertion hole communicating with the second channel 233b. The first end part 241 of the tube 240 is inserted into the fixing part 234 and fitted thereinto, and thereby is fixed with respect to the puncture part 230.

As illustrated in Fig. 3, the projecting direction of the needle tube 220 from the contact surface 231 is substantially perpendicular to the contact surface 231. Thus, the axis of the needle tube 220 intersects the contact surface 231 substantially perpendicularly.

As illustrated in Fig. 3, the puncture part 230 of the administering instrument 200 has a thickness (dimension in the horizontal direction illustrated in Fig. 3) t1 smaller than a thickness t2 of the housing 120 of the medicinal solution administering device 100. It should be noted that, in a state where the puncture part 230 is placed on a horizontal plane, a thickness t1 of the puncture part 230 is a dimension of a portion having the largest thickness of the puncture part 230. Similarly, in a state where the housing 120 is placed on a horizontal plane, the thickness t2 of the housing 120 is a dimension of a portion having the largest thickness of the housing 120.

The puncture part 230 can be formed to have a thickness t1 of 3 mm to 30 mm, for example. Further, the housing 120 can be formed to have a thickness t2 of 10 mm to 30 mm, for example.

As with the medicinal solution administering device 100, the administering instrument 200 is configured as a patch type instrument adhered to the body surface H of the user and used. The puncture part 230 of the administering instrument 200 has, on a contact surface (bottom surface) 231, a sheet-like adhesive part (not shown) that can be adhered to the body surface. In the initial state before the administering instrument 200 is attached to the user, a peelable protective sheet is attached to an adhesive surface of the adhesive part.

In a case where the administering instrument 200 punctures the living body with the puncture auxiliary tool 250 attached to the puncture part 230 and the tip of the introducer needle 251 projecting from the tip of the needle tube 220, the contact surface 231 of the puncture part 230 is adhered to the body surface of the user. The puncture auxiliary tool 250 is detached after the needle tube 220 punctures the living body, and the puncture part 230 remains on the body surface H of the user in a state where the needle tube 220 punctures the living body. In this state, the plunger 130 of the medicinal solution administering device 100 moves forward in the medicinal solution container 110, which allows the medicinal solution filled in the lumen 111 of the medicinal solution container 110 of the medicinal solution administering device 100 to be fed to the lumen of the needle tube 220 via the communication part 211 of the connector 210, the tube 240, and the communication passage 233 of the puncture part 230.

As described above, the administering instrument 200 according to the present embodiment includes the connector 210 that includes the communication part 211 capable of communicating with the lumen 111 of the medicinal solution container 110 and is connectable to the medicinal solution administering device 100, the needle tube 220 that punctures the living body, the puncture part 230 including the contact surface 231 that is provided in a lower end to contact the body surface H of the living body, the needle holding part 232 that is provided above the contact surface 231 to hold the needle tube 220 so that a tip of the needle tube 220 projects from the contact surface 231, and the communication passage 233 communicating with the lumen of the needle tube 220; and the tube 240 that includes the first end part 241 connected to the puncture part 230, the second end part 242 connected to the connector 210, and the tube body 243 communicating from the first end part 241 to the second end part 242 and is configured to supply the medicinal solution from the lumen 111 of the medicinal solution container 110 to the needle tube 220 via the communication part 211 of the connector 210 and the communication passage 233 of the puncture part 230. The tube body 243 has a total length longer than the straight-line distance d1 between the first end part 241 and the second end part 242.

The administering instrument 200 is sometimes disposed in such a manner that, for example, in a state where the puncture part 230 and the housing 120 are attached to the curved body surface H of the user and so on, the position of the contact surface 121 of the housing 120 is higher than the position of the contact surface 231 of the puncture part 230 (the distance away from the body surface H is larger in the former than in the latter). Particularly, in a case where the puncture part 230 is formed to have a thin shape in consideration of convenience at the use of the administering instrument 200 (such as avoiding the interference of the administering instrument 200 attached to the body surface H), the thickness t1 of the puncture part 230 is smaller than the thickness t2 of the housing 120; therefore, the deviation in the height direction between the contact surface 231 of the puncture part 230 and the contact surface 121 of the housing 120 is significant. When the user moves such as twisting his/her body, due to the deviation in the height direction at the time of attachment to the body surface H as described above, a force that lifts the puncture part 230 from the medicinal solution administering device 100 side toward the puncture part 230 acts via the first end part 241 of the tube 240 fixed to the puncture part 230. This may remove the puncture part 230 from the body surface H and the needle tube 220 held by the puncture part 230 may come out of the living body.

In light of the foregoing issues, in the administering instrument 200 according to the present embodiment, the tube body 243 has a total length longer than the straight-line distance d1 between the first end part 241 and the second end part 242. In a case where a force acting on the puncture part 230 from the medicinal solution administering device 100 side via the tube 240 (force that lifts the puncture part 230) is to develop, the tube 240 absorbs (reduces) the force by deforming a part of the tube body 243. This allows the administering instrument 200 to prevent the force from being conveyed to the puncture part 230 via the tube 240. Therefore, the administering instrument 200 can suitably prevent the needle tube 220 from coming out of the living body during administration of the medicinal solution. Particularly, even in a case where the tube 240 is made of a material inferior in flexibility to other resin materials such as polybutadiene, the administering instrument 200 can suitably prevent the needle tube 220 from coming out of the living body during administration of the medicinal solution.

Further, in the administering instrument 200, at least a part of the tube body 243 extends in a bellows shape. This enables the tube body 243 to absorb the force that lifts the puncture part 230 at the part formed in the shape of a bellows.

Further, the medicinal solution administering system 10 according to the present embodiment includes the administering instrument 200 and the medicinal solution administering device 100 that has the medicinal solution container 110, the housing 120 for holding the medicinal solution container 110 therein, the plunger 130 for pushing out the medicinal solution in the medicinal solution container 110 to the tube 240. With the use of the medicinal solution administering system 10, it is possible to absorb the force acting on the puncture part 230 (force that lifts the puncture part 230) at the tube body 243 of the tube 240 during administration of the medicinal solution. Therefore, the medicinal solution administering system 10 can suitably prevent the needle tube 220 held by the puncture part 230 from coming out of the living body.

Further, in the medicinal solution administering system 10, the thickness t1 of the puncture part 230 of the administering instrument 200 is smaller than the thickness t2 of the housing 120 of the medicinal solution administering device 100. According to the medicinal solution administering system 10, it is therefore possible to reduce the thickness of the puncture part 230 and suitably prevent the occurrence of a problem that the needle tube 220 held in the puncture part 230 comes out of the living body due to the reduction in thickness of the puncture part 230.

### Second Embodiment

The description goes on to an administering instrument 200A according to the second embodiment. The same configurations as those in the first embodiment are denoted by the same reference numerals and description thereof is omitted. In addition, configurations not particularly mentioned in the second embodiment can be configured in substantially the same manner as described above in the first embodiment.

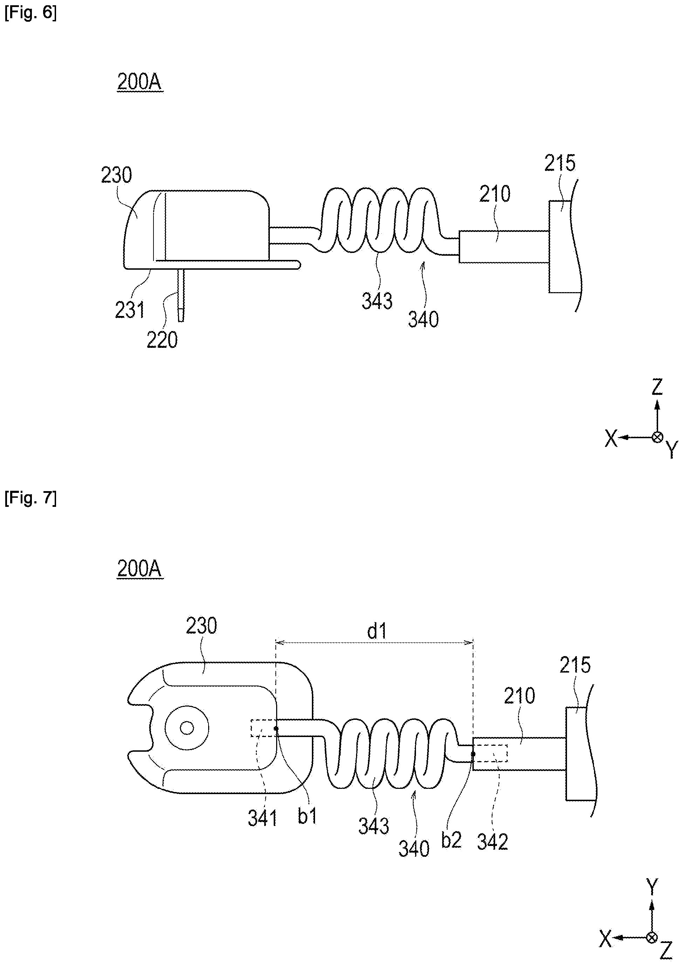

Figs. 6 and 7 are diagrams illustrating the administering instrument 200A according to the second embodiment.

The administering instrument 200 according to the first embodiment is so formed that at least a part of the tube body 243 of the tube 240 extends in a bellows shape (see Fig. 5). On the other hand, in the administering instrument 200A according to the second embodiment, at least a part of a tube body 343 of a tube 340 extends in a spiral shape as illustrated in Figs. 6 and 7.

The tube body 343 has a total length longer than a straight-line distance d1 between a first end part 341 and a second end part 342.

The "spiral" shape according to the present embodiment means a shape of the tube 340 that winds three-dimensionally around an axis of the tube 240 straightened out. In other words, the tube 340 has a shape in which at least a part of the tube body 343 is bent in a spiral shape in the X-Y-Z direction.

The length of the spiral part of the tube body 343, the number of repetitions of spiral in the direction in which the tube body 343 extends, the position at which the spiral part of the tube body 343 is formed, and so on are not particularly limited, and can be modified suitably.

The length of the tube body 343 means a length of the entirety of the tube 340 stretched to a nearly straight shape (the maximum length of the tube body 343). Further, the straight-line distance d1 between the first end part 341 and the second end part 342 corresponds to, as illustrated in Fig. 7, a straight-line distance between a boundary b1 (an end on the first end part 341 side of the tube body 343) between the first end part 341 and the tube body 343 and a boundary b2 (an end on the second end part 342 side of the tube body 343) between the second end part 342 and the tube body 343.

The tube 340 can be formed to have a total length (length in a state where the first end part 341, the second end part 342, and the tube body 343 are straightened out) of, for example, 20 mm to 200 mm, the first end part 341 of the tube 340 (length of a part inserted into the puncture part 230) can be formed to be, for example, 2 mm to 20 mm, the second end part 342 of the tube 340 (length of a part inserted into the connector 210) can be formed to be, for example, 2 mm to 20 mm, and the tube body 343 of the tube 340 can be formed to have a total length (length in a state where the tube body 243 is straightened out) of, for example, 15 mm to 196 mm. Further, a part with a spiral shape of the tube body 343 can be formed to have a length of, for example, 15 mm to 195 mm in a state where the part is straightened out.

The straight-line distance d1 between the first end part 341 and the second end part 342 can be 10 mm to 150 mm, for example. The total length of the tube body 343 is preferably 1.3 to 10 times longer than the straight-line distance d1.

As described above, in the administering instrument 200A according to the present embodiment, at least a part of the tube body 343 of the tube 340 extends in a spiral shape. Therefore, in the administering instrument 200A, as with the administering instrument 200 according to the first embodiment, in a case where a force acting on the puncture part 230 from the medicinal solution administering device 100 side via the tube 340 (force that lifts the puncture part 230) is to develop, a part of the tube body 343 deforms to absorb (reduce) the force. Thus, the administering instrument 200A can suitably prevent the needle tube 220 from coming out of the living body during administration of the medicinal solution.

Further, since at least a part of the tube body 343 of the tube 340 has a spiral shape, in a case where a force that lifts the puncture part 230 is to develop, the tube body 343 deforms three-dimensionally to absorb the force, which further prevents the puncture part 230 from being lifted. Further, since at least a part of the tube body 343 flexes to have a three-dimensional spiral shape, even in a case where the tube body 343 has an elongated part that absorbs the force, it is possible to prevent an excessive long distance between the first end part 341 and the second end part 342. Thus, the administering instrument 200A can be made more compact.

The administering instrument and the medicinal solution administering system according to the embodiments of the present invention are describe above. The present invention is not limited only to the configurations described above, and can be modified suitably on the basis of the description of claims.

For example, the tube body may both have the bellows shape described in the first embodiment and the spiral shape described in the second embodiment. Further, the shape of the tube body is not particularly limited as long as the tube body has a total length longer than the straight-line distance between the first end part and the second end part to absorb (reduce) the force that lifts the puncture part. The tube body can be configured to have, for example, a shape other than the bellows or spiral shape.

In addition, the material, shape, size, and arrangement of the members constituting the administering instrument and the medicinal solution administering system, and the connecting/coupling structures of the members thereof are not particularly limited as long as the effects of the present invention are exhibited, and can be arbitrarily changed and replaced. Moreover, it is also possible to appropriately add any constituent members or the like, which are not particularly described in the specification, to the administering instrument and the medicinal solution administering system.

This application is on the basis of Japanese Patent Application No. 2018-067426 filed on March 30, 2018, the disclosure of which is incorporated by reference in its entirety.

### Reference Signs List

10 Medicinal solution administering system
100 Medicinal solution administering device
110 Medicinal solution container
111 Lumen
120 Housing
121 Contact surface of housing
130 Plunger
200, 200A Administering instrument
210 Connector
211 Communication part
215 Mounting part
220 Needle tube
230 Puncture part
231 Contact surface of puncture part
232 Needle holding part
233 Communication passage
234 Fixing part
240, 340 Tube
241, 341 First end part
242, 342 Second end part
243, 343 Tube body
250 Puncture auxiliary tool
d1 Straight-line distance between first end part and second end part
t1 Thickness of puncture part
t2 Thickness of housing
H Body surface

## Claims

1. An administering instrument connectable to a medicinal solution administering device including a medicinal solution container in which a medicinal solution is filled, the administering instrument comprising:
a connector that includes a communication part capable of communicating with a lumen of the medicinal solution container and is connectable to the medicinal solution administering device;
a needle tube that punctures a living body;
a puncture part including a contact surface that is provided in a lower end to contact a living body surface of the living body, a needle holding part that is provided above the contact surface to hold the needle tube so that a tip of the needle tube projects from the contact surface, and a communication passage communicating with a lumen of the needle tube; and
a tube that includes a first end part connected to the puncture part, a second end part connected to the connector, and a tube body communicating from the first end part to the second end part, and is configured to supply the medicinal solution from the lumen of the medicinal solution container to the needle tube via the communication part of the connector and the communication passage of the puncture part, wherein
the tube body has a total length longer than a straight-line distance between the first end part and the second end part.

2. The administering instrument according to claim 1, wherein at least a part of the tube body extends in a bellows shape.

3. The administering instrument according to claim 1 or 2, wherein at least a part of the tube body extends in a spiral shape.

4. The administering instrument according to any one of claims 1 to 3, wherein the total length of the tube body is 1.3 to 10 times longer than the straight-line distance between the first end part and the second end part.

5. A medicinal solution administering system comprising:
the administering instrument according to any one of claims 1 to 4; and
the medicinal solution administering device including the medicinal solution container, a housing that holds the medicinal solution container, and a plunger that pushes out the medicinal solution in the medicinal solution container to the tube.

6. The medicinal solution administering system according to claim 5, wherein the puncture part of the administering instrument has a thickness smaller than a thickness of the housing of the medicinal solution administering device.
